# EUROPÄISCHE PATENTANMELDUNG

(11) **EP 0 776 906 A2**
(43) Veröffentlichungstag der Anmeldung: **04.06.1997**
(21) Anmeldenummer: 96250229.0
(22) Anmeldetag: 14.10.1996
(51) Int. Cl.: C07K 14/47, C07K 16/18, C07K 16/42, G01N 33/68, A61K 38/17, A61K 39/385

(54) **Peptide des Antigens Sm-D und ihre Verwendung, insbesondere für die Diagnostik des SLE**

(30) Priorität: 19.10.1995 DE 19538968; 14.05.1996 DE 19619418
(71) Anmelder: IMTEC IMMUNDIAGNOSTIKA GMBH, 16341 Zepernick (DE)
(72) Erfinder: Hiepe, Falk, Dr. med., 12557 Berlin (DE); Riemekasten, Gabriele, Dr. med., 12689 Berlin (DE); Marell, Jeannette, 13156 Berlin (DE); Burmester, Gerd-Rüdiger, 12277 Berlin (DE)
(74) Vertreter: Baumbach, Friedrich, Dr.

(57) **Zusammenfassung**

Die Erfindung betrifft Peptide des Antigens Sm-D, bestehend aus 35 bis 45 Aminosäuren, die ein Konformationsepitop bilden und Autoantikörper binden können, wie sie beim systemischen Lupus erythematodes (SLE) vorkommen.
Besonders bevorzugt ist das Peptid aus 37 Amonisäuren mit der Struktur VEPKVKSKKREAVAGRGRGRGRGRGRGRGRGRGGPRR bzw. seine Mutanten und Varianten.

## Beschreibung

Die Erfindung betrifft Peptide des Antigens Sm-D, die von Antikörpern in biologischen Flüssigkeiten erkannt werden, insbesondere von Antikörpern, die in Körperflüssigkeiten von Patienten auftreten, die am systemischen Lupus erythematodes (SLE) erkrankt sind.

Weiterhin betrifft die Erfindung die Verwendung dieser Peptide und ihrer Folgeprodukte für die Diagnostik von SLE in vitro, sowie ihre Verwendung als Vakzine zur Erzeugung von Toleranzmechanismen und die Bereitstellung immunogener und antigener Kompositionen und von Testkits.

Außerdem betrifft sie Antikörper, die durch diese Peptide in vivo induziert werden sowie ein Verfahren zur Bestimmung von Anti-Sm-D-Antikörpern in biologischen Flüssigkeiten.

Es ist bekannt, daß viele Autoantigene in enger räumlicher Beziehung zueinander stehen. So bilden z. B. die Sm- und die verschiedenen RNP-Proteine einen Ribonukleoproteinkomplex, der im Kern eukarioter Zellen vorhanden ist (snRNP). Anti Sm-Antikörper erkennen verschiedene Proteine des snRNP-Komplexes, die als B', B, D, E, F und G bezeichnet werden. Dieser Komplex spielt wahrscheinlich eine zentrale Rolle beim Splicen der prä-mRNA, wobei das Sm-D-Protein ein wichtiges Regulationsprotein darstellt, indem es die Bindung verschiedener RNA's regulieren soll.

Welche Mechanismen zur Autoantikörperbildung führen, ist letztlich noch ungeklärt. Auffällig ist, daS für den Zellzyklus besonders wichtige Regulationszentren Ziel der Autoantikörper beim SLE und ihm verwandten syndromen sind. Bei der Entstehung von Autoimmunerkrankungen sollen im allgemeinen besonders wichtige Regionen von Autoantigenen erkannt werden und ihre physiologische Funktion stören.

Bloom und Mitarbeiter konnten an Untersuchungen vom Hybridomzellinien nachweisen, daß mittels Hybridomtechnik produzierte Anti-Sm-Ak auch DNA erkennen können (Bloom, D.D. et al, J. Immunol. 1993; 150 (4): 1579-1590). Die Autoren schlußfolgerten daraus, daß für die Bildung von Anti-Sm-Ak nicht das Sm-D-Protein allein als Antigen wirkt. Vielmehr wird angenommen, daß sowohl das Sm-D-Autoantigen als auch die DNA - möglicherweise als Komplex - das autoimmune Agens bilden. Bisher konnte noch keine Bindungsfähigkeit des Sm-D-Proteins mit der DNA festgestellt werden. Die physiologische und pathophysiologische Bedeutung des Sm/DNA-Komplexes ist bisher nicht bekannt.

Lebrun und Mitarbeiter führten Immunisierungsversuche an bestimmten Mäusestämmen mit DNA durch, die sie mit einem argininreichen Fusionsprotein koppelten und dadurch eine Lupusnephritis erzeugen konnten (Lebrun, P. et al, Lupus 1994; 3: 47-53). Auch sie schlußfolgerten, daß bestimmte, an die DNA-bindende Proteine notwending sind, um eine autoimmune Reaktion zu erzeugen.

Für den systemischen Lupus erythematodes (SLE) gelten neben den Antikörpern (Ak) gegen native oder Doppelstrang-DNA (dsDNA) Anti-Sm-Ak als diagnostischer Marker. Außerdem wird ihnen eine pathogenetische Rolle bei der Entstehung von Organschädigungen zugeschrieben. Der Nachweis der Anti-Sm-Ak gelingt in Europa (im Gegensatz zu den Anti-dsDNA-Ak) nur bei relativ wenigen Patienten, während sie in den USA bei ca. einem Drittel der Patienten mit SLE bestimmt werden können. Als Ursache wird eine andere ethnische Zusammensetzung der Bevölkerung angesehen. (Abuaf, N. et al, Eur. J. Clin. Invest. 1990; 20: 354-359).

Es gibt verschiedene Nachweisverfahren für die Bestimmung von Anti-Sm-Ak.

Relativ spezifische Verfahren für den Nachweis von Anti-Sm-Ak sind die Immundiffussion und Gegenstromelektrophorese, jedoch sind diese wenig sensitiv und zudem zeitaufwendig und teuer.

Mittels Immunoblotting können Anti-Sm-Ak je nach dem verwendeten Antigensubstrat auch in Europa in ca. 30 % der SLE-Patienten nachgewiesen werden (Riemekasten, G. und F. Hiepe, Z. ärztl. Fortbild. 1992; 86: 217-222). Durch dieses Verfahren ist es möglich, Anti-Sm-Ak besser zu charakterisieren. So reagieren Anti-Sm-Ak-positive Patienten u.a. mit den B'B-Proteinen sowie mit den Proteinen D,E,F und G eines kleinen nukleären Ribonukleoproteinkomplexes (snRNP). Während Anti-B'B-Ak nicht so spezifisch für den SLE sind, gelten Antikörper insbesondere gegen das D-Protein als SLE-spezifisch.

Diese Immunoblotting-Methode hat jedoch den Nachteil, daß sie einen erheblichen Zeitaufwand erfordert und teuer ist. Darüber hinaus wird viel Erfahrung bei der Auswertung benötigt.

Es werden deshalb in vielen Laboratorien kommerziell erhältliche ELISA-Techniken für den Nachweis von Sm-Ak verwendet, die sich durch eine hohe Sensitivität und durch eine relativ einfache Durchführbarkeit bei hohem Probendurchsatz auszeichnen. Diese Tests werden beispielsweise auf der Basis von aus Thymusextrakten gereinigtem Sm als Festphasen-Antigen (WO 90/10229) und rekombinant hergestelltem Sm-Antigen (Rokeach, L.A. et al, Clin. Immunol. and Immunopathol. 1992; 65(3): 315-324) durchgeführt. Rokeach und Mitarbeiter konnten erstmals die cDNA des Sm-D-Proteins isolieren und zeigen, daß sie ein aus 119 AS bestehendes Protein kodiert.

Damit waren die Grundlagen für die Entwicklung von ELISA's auf der Basis von synthetisierten Peptiden geschaffen, vgl. EP 0 295 719, in dem die Anwendung von kloniertem Sm-D zur Diagnostik des SLE beschrieben wurde.

Eine Vielzahl dieser z.T. auch kommerziell erhältlichen Tests sind jedoch relativ instabil, zeitaufwendig und aufgrund von falsch positiven Ergebnissen infolge von Kreuzreaktionen weniger spezifisch (WO 90/10229).

Um die Hauptepitope des Sm-D-Proteins zu ermitteln, wurden von James und Mitarbeitern das ganze SmD-Protein in überlappende Oktapetide zerlegt und die Reaktivitäten im ELISA gegenüber 15 SLE-Seren untersucht (James et al, Clin. exp. Immunol. 1994; 98: 419-426). Es wurden 5 Epitope gefunden, Hauptepitope waren ebenfalls am C-terminalen Ende sowie Epitope der AS 37-53, der AS 69-76 und der AS 5-12. Barakat und Mitarbeiter fanden 3 Regionen des SmD-Proteins (Region 1. bis 20. AS, 44. bis 67. AS und 97. bis zur 119. AS) mit denen zuvor im Immunoblotting nachgewiesene Anti-SmD-Ak-positive Seren reagieren (Barakat et al, Clin. exp. Immunol. 1990; 81: 256-262). 67 % der insgesamt 18 Anti-SmD-Ak-positiven Seren reagierten mit dem Peptid 1-20, 89 % mit dem Peptid 44-67 und nur 33 % mit dem Peptid 97-119. 165 SLE-Seren wurden hinsichtlich ihrer Reaktivität im ELISA mit den Peptiden 1-20, 44-67 und 97-119 getestet, wobei positive Reaktionen in 59 %, 37 % und 1 % auftraten. Sabbatini und Mitarbeiter fanden positive Reaktionen gegen die Region 95-119 in 25 % der 48 SLE-Seren im ELISA ( Sabbatini, A. et al, J. Rheumatol. 1993; 20: 1679-1683).

Bei all diesen Untersuchungen wurden kurzkettige Peptide verwendet, und die Anzahl der positiven Resultate bei SLE-Seren schwankte für das C-terminale Ende zwischen 1 % und 33 % im ELISA. Damit bringen diese synthetisch hergestellten Peptide gegenüber den kommerziell erhältlichen Tests keinen wesentlichen Vorteil. Mit diesem Epitopmapping können nur lineare Epitope erfaßt werden. Die Reaktivität bei Autoimmunerkrankungen ist jedoch gegen intakte Autoantigene gerichtet ( Tan, E.M., J. Exp. Med. 1994; 179: 1083-1086).

Bei einer möglichen Verwendung der gesamten Sm-D-Antigene besteht die Gefahr, daß wichtige Epitope durch Sekundär- und Tertiärstruktur für die Antikörper nicht mehr zugänglich sind. Außerdem kann durch Bindung von Teilen des Sm-D-Antigens an andere Autoantigene, vorzugsweise DNA, eine neue Konformation entstehen.

Ein Nachweis einer Reaktivität der Anti-La-Ak , die gegen ein Konformationsepitop gerichtet ist, konnte mittels verschieden langer Peptidketten des La-Proteins als Festphasenantigen im ELISA durch Rischmueller und Mitarbeiter erbracht werden (Rischmueller, M. et al, Clin. exp. Immunol. 1995; 101: 39-44).

In WO 91/18920 sind Peptide des Sm-D-Antigens und ihre Verwendung für die Diagnostik des SLE beschrieben. Diese bekannten Peptide, deren Aminosäuren haupsächlich N-terminal orientiert sind, weisen jedoch nur eine niedrige Frequenz positiver Reaktionen auf (ca. 30 % positive Reaktionen).

Der Erfindung lag deshalb die Aufgabe zugrunde, spezifische Peptide des Antigens Sm-D bereitzustellen, wobei diese Peptide und ihre Folgeprodukte für die Diagnose von SLE besonders gut geeignet sein sollen.
Desweiteren lag der Erfindung die Aufgabe zugrunde, auf der Basis dieser Peptide einen einfachen, schnell durchführbaren und sicheren Test zur Diagnose von SLE zu entwickeln, d.h. den Prozentsatz von echt-positiven zu falsch-positiven Resultaten zu senken.
Außerdem besteht die Aufgabe darin, Antikörper und Anti-Idiotyp-Antikörper bilden oder binden zu können, die in ihrer Spezifität den Autoantikörpern von SLE-Patienten entsprechen.

Die Aufgabe wird erfindungsgemäß durch die Merkmale der Ansprüche realisiert.

Überraschend wurden solche Peptide, die ein Konformationsepitop bilden und Autoantikörper binden können, wie sie beim systemischen Lupus erythematodes (SLE) vorkommen, in Peptiden des Proteins Sm-D mit 35 bis 45 Aminosäuresequenzen gefunden.

Bevorzugt handelt es sich um die Peptide
a) PKVKSKKREAVAGRGRGRGRGRGRGRGRGRGGPRR
b) EPKVKSKKREAVAGRGRGRGRGRGRGRGRGRGGPRR
c) VEPKVKSKKREAVAGRGRGRGRGRGRGRGRGRGGPRR
d) DVEPKVKSKKREAVAGRGRGRGRGRGRGRGRGRGGPRR
e) VDVEPKVKSKKREAVAGRGRGRGRGRGRGRGRGRGGPRR
f) RVDVEPKVKSKKREAVAGRGRGRGRGRGRGRGRGRGGPRR
g) IRVDVEPKVKSKKREAVAGRGRGRGRGRGRGRGRGRGGPRR
h) TIRVDVEPKVKSKKREAVAGRGRGRGRGRGRGRGRGRGGPRR
i) DTIRVDVEPKVKSKKREAVAGRGRGRGRGRGRGRGRGRGGPRR
j) LDTIRVDVEPKVKSKKREAVAGRGRGRGRGRGRGRGRGRGGPRR
k) PLDTIRVDVEPKVKSKKREAVAGRGRGRGRGRGRGRGRGRGGPRR.

Besonders bevorzugt ist das Peptid aus 37 Aminosäuren mit der Struktur
VEPKVKSKKREAVAGRGRGRGRGRGRGRGRGRGGPRR,
bzw. seine Mutanten und Varianten, nämlich durch die an einer oder mehreren beliebigen Stellen durch Austausch von Aminosäuren geänderte Struktur bzw. durch die an einer oder mehreren beliebigen Stellen durch Entfernen von Aminosäuren geänderte Struktur.

Die Synthese der Peptide erfolgt nach an sich üblichen Verfahren (Atheron, E. and R. C. Sheppard, In Solide Phase Synthesis - A Practical Approach, IRL Press at Oxford University Press, 1989).

Die Spezifitäts- und Aktivitätsprüfung dieser Peptide wird mittels "Enzyme-linked immunsorbent assay" (ELISA) durchgeführt.

Erfindungsgemäße Antikörper, die gegen Konformationsepitope des Sm-D-Antigens gerichtet sind, sind dadurch gekennzeichnet, daß sie ein oder mehrere der erfindungsgemäßen Peptide des Antigens Sm-D erkennen können, insbesondere die oben genannten Peptide a) bis k) bzw. deren Mutanten und Varianten. Ihre Herstellung erfolgt nach an sich üblichen Verfahren durch
- Immunisierung von Mäusen oder Immunisierung von Milzzellen in vitro mit den erfindungsgemäßen Peptiden
- Isolierung der Milzzellen und Fusionierung mit Krebszellen zu Hybridomzellen; Selektion positiver Klone
- Isolierung der Antikörper.

Diese Antikörper werden sowohl in verschiedenen immunologischen Testsystemen, in unterschiedlichen ELISA-Testsystemen, in der Durchflußcytometrie und im Western Blot eingesetzt.

Erfindungsgemäße Anti-Idiotyp-Antikörper, welche gegen Antikörper gebildet werden, die gegen Konformationsepitope des Sm-D-Antigens gerichtet sind und die die erfindungsgemäßen Peptide erkennen, sind dadurch charakterisiert, daß sie auf spezifische Art die Autoantikörper des SLE in biologischen Flüssigkeiten erkennen. Ihre Herstellung erfolgt ebenfalls nach an sich bekannten Verfahren durch Immunisierung mit den entsprechenden Autoantikörpern.

Antigene Kompositionen enthalten erfindungsgemäß ein oder mehrere dieser erfindungsgemäßen Peptide, bevorzugt das Peptid VEPKVKSKKREAVAGRGRGRGRGRGRGRGRGRGGPRR, bzw. seine Mutanten und Varianten, und sind dadurch charakterisiert, daß sie mit den Autoantikörpern, wie sie in biologischen Flüssigkeiten beim SLE vorkommen, spezifisch reagieren und sind ggf. an verschiedene Träger, wie Polystyren, chemisch aktiviertes Polystyren, aktivierte Sepharosen, Zellulosen und ähnliche gekoppelt, die die Antigenität noch verbessern können.

Immunogene Kompositionen enthalten ein erfindungsgemäßes Peptid oder eine Verbindung dieses Peptids mit einem Trägermolekül oder einem anderen Auto-Ag, vorzugsweise DNA, bevorzugt das Peptid
VEPKVKSKKREAVAGRGRGRGRGRGRGRGRGRGGPRR, bzw. seine Mutanten und Varianten, und induzieren die Produktion der Antikörper, die fähig sind, Autoantigene beim SLE zu erkennen.

Das erfindungsgemäße Verfahren zur Bestimmung von Anti-Sm-D-Antikörpern in biologischen Flüssigkeiten wird durchgeführt, indem mindestens eines der erfindungsgemäßen Peptide, das ggf. an ein Trägermaterial gekoppelt ist, oder auch ein Anti-Idiotyp-Antikörper mit den biologischen Flüssigkeiten unter Bedingungen, die eine Antigen-Antikörper-Reaktion zulassen, in Kontakt gebracht wird und der Nachweis dieser Antigen-Antikörper-Reaktion durch physikalische oder chemische Methoden erfolgt.

Gemäß der Erfindung wird außerdem ein Testkit für die Bestimmung von Anti-Sm-D-Antikörpern zur Verfügung gestellt. Dieser Testkit ist gekennzeichnet, durch
- mindestens ein erfindungsgemäßes Peptid oder einer Verbindung dieses Peptids mit einem Trägermolekül, bevorzugt das Peptid VEPKVKSKKREAVAGRGRGRGRGRGRGRGRGRGGPRR, bzw. seine Mutanten und Varianten, oder auch ein erfindungsgemäßer Anti-Idiotyp-AK, der adsorptiv oder kovalent an eine feste Phase gebunden ist,
- einen Puffer zur Verdünnung der zu untersuchenden biologischen Flüssigkeit, der nicht-ionische oder ionische Detergentien oder Proteine enthält,
- ein spezifisches Konjugat, bestehend aus einem Antikörper, der gegen humanes Immunglobulin IgG, IgM, IgA oder gegen mehrere dieser Immunglobuline gerichtet ist, und einem Enzym,
- einen Puffer zum Verdünnen des Konjugats, der nicht-ionische oder ionische Detergentien oder Proteine enthält,
- einen Puffer zum Waschen (Entfernen) der nichtgebundenen Immunreaktanten, der nicht-ionische oder ionische Detergentien oder Proteine enthält,
- eine Substratlösung zum Nachweis der Enzymreaktion und
- eine Stopp-Lösung zum Unterbrechen der Enzymreaktion.

Mit der Entwicklung dieses neuen Testkits auf der Basis der erfindungsgemäßen Peptide lassen sich Verlaufsbeurteilungen relativ einfach und schnell durchführen.

Mit dem erfindungsgemäßen Test, der ein 35-45 Aminosäuren langes Peptid des Sm-D an der festen Phase enthält, lassen sich Anti-Sm-Ak mindestens ebenso häufig nachweisen wie Anti-dsDNA-Ak. Die Titer der Antikörper korrelieren gut mit der Aktivität des SLE, so daß dieser Test neben den Krankheitssymptomen die Verlaufsbeurteilung und Einschätzung des Therapieerfolges ermöglicht. Somit können aufwendige Tests ersetzt werden.

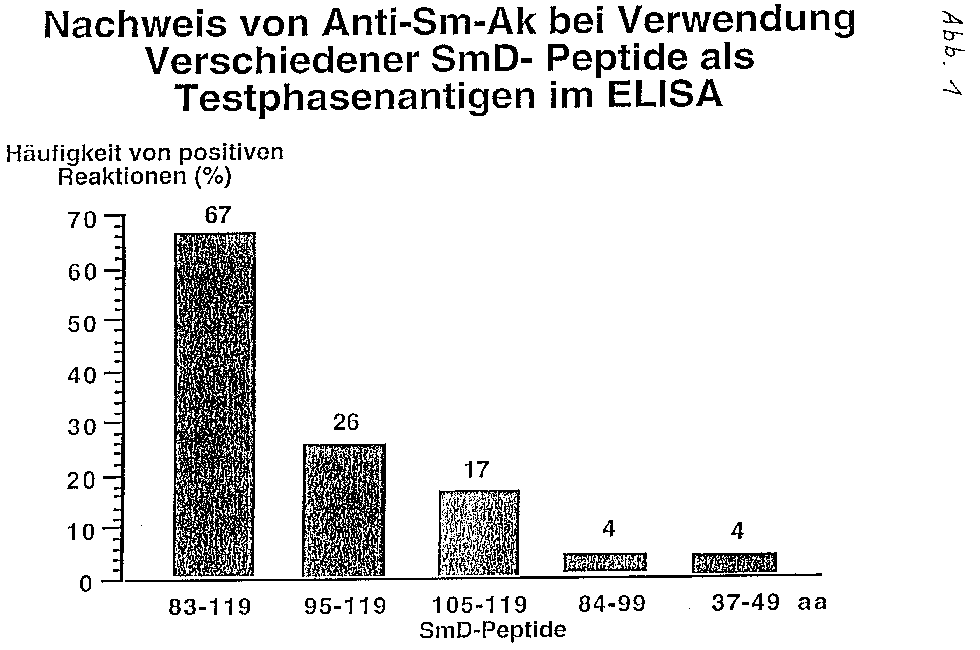

Überraschend weisen die erfindungsgemäßen Peptide eine außergewöhnlich hohe Frequenz von positiven Reaktionen auf. Sie liegt z.B. für das Peptid
VEPKVKSKKREAVAGRGRGRGRGRGRGRGRGRGGPRR bei über 67 %, wodurch analoge bekannte Peptide (positive Reaktionen bei ca. 30 %) deutlich übertroffen werden (vgl. Abb. 1).

Durch die Erfindung kann die Diagnose eines SLE mit hoher Sicherheit gestellt werden. Die Erfindung ist jedoch nicht nur für die Diagnose des SLE, sondern darüber hinaus auch für eine Therapiekontrolle (Monitoring) sowie zur Produktion von Medikamenten gegen SLE hervorragend geeignet.

Außerdem können die erfindungsgemäßen Peptide und ihre Folgeprodukte als Vakzine zur Erzeugung von Toleranzmechanismen verwendet werden.

Anschließend wird die Erfindung an Beispielen näher erläutert, die die Erfindung aber nicht auf diese beschränken sollen.

### Beispiel 1

Um ein Konformationsepitop zu erfassen, wurden 4 verschiedene Peptide des Sm-D-Proteins synthetisiert.
1. Peptid 83-99 mit folgender Sequenz: VEPKVKSKKREAVAGRG
2. Peptid 95-119 mit folgender Sequenz: VAGRGRGRGRGRGRGRGRGRGGPRP
3. Peptid 105-119 mit folgender Sequenz: GRGRGRGRGRGGPRP
4. Peptid 83-119 mit folgender Sequenz: VEPKVKSKKREAVAGRGRGRGRGRGRGRGRGRGGPRP

Die Synthese der Peptide erfolgte nach der Methode, die erstmals von Atherton (vgl. oben) beschrieben wurde. Zur Reinigung der Peptide wurde eine 300 x 40 mm VYDAC TPB 1520 300A C18 Revers phase-Säule benutzt. Die Fluß-Rate betrug 100 ml/min.

Diese Peptide wurden mit einem ELISA getestet.

### Herstellung des ELISA

### Beschichtung der Antigene (Peptide) an die feste Phase des ELISAs

Für die Beschichtung wurden Polystyren-Platten (Nunc, Dänemark) mit je 96 Wells verwendet. Die Beschichtung erfolgte mit 10 µg/ml, wobei 50 µl je Weil verwendet wurden. Als Benetzungsmedium diente 0,05 M Carbonatpuffer, pH 9,5.

Die Benetzung erfolgte über Nacht bei Raumtemperatur. Nach dreimaligem Waschen mit dem Waschpuffer (PBS-Tween 0,1%, pH 7,2) wurden die Platten entweder weiter verwendet oder bei - 20°C eingefroren.

Vor Wiederverwendung nach dem Einfrieren wurden die Platten erneut 3x gewaschen.

### Probenauftragung von 40 SLE-Seren sowie von 50 Seren gesunder Probanden

Vor dem Auftragen der Proben erfolgt eine Vorblockierung der Platten mit je 100 µl/Well Probenpuffer (PBS-Tween, pH 7,2, im Verhältnis 1:100 mit Trockenmilch) für eine Stunde bei Raumtemperatur. Ohne einen Waschvorgang anzuschließen, erfolgt die Probenauftragung (Serumverdünnung 1:100 in Probenpuffer) mit je 50 µl/Well über 2 Stunden bei Raumtemperatur. Anschließend erfolgt 3x der Waschvorgang mit dem Waschpuffer.

### Konjugatzugabe

Die Inkubation mit POD-markiertem Anti-Human-IgG erfolgt in einer Verdünnung von 1:1000 in Probenpuffer jeweils 50 µl/Well über 2 Stunden bei Raumtemperatur. Anschließend wird wiederum 3x mit dem Waschpuffer gewaschen.

### Färbung

Die Färbung der Antigen-Antikörperreaktion erfolgt mit 10 ml Citratpuffer, pH 5,0, 5 µl H₂O₂ und 5 mg Orthophenylendiamin (OPD) jeweils 50 µl/Well über 1-3 Minuten. Das Stoppen erfolgt mit 150 µl/Well Stopplösung (100 ml 1M H₂SO₄, 0,36 mg Natriumsulfit). Die Farbintensität oder optische Dichte (Extinktionen) wurde bei 495 mm gemessen. Wie bei jedem anderen ELISA ist die resultierende Farbe der Reaktion proportional der Anzahl der konjugierten Antikörper, welche am Testantikörper gebunden wurden. In den meisten Fällen ist die Anzahl der gebundenen konjugierten Antikörper linear bezogen auf die Anzahl der an das Antigen gebundenen Antikörper.

Es wurde ein Standardserum zur Erstellung einer Eichkurve verwendet, welches in Vorversuchen besonders gut reagierte. Die Konzentration dieses Standardserums in einer Verdünnung von 1:100 wurde mit 10 000 Einheiten definiert. Diese Eichkurve wird auf jede Platte aufgetragen, und jedes Serum wurde doppelt bestimmt.

Der Normbereich (NB) wurde definiert mit: NB = Mittelwert der Normalseren plus dem zweifachen Wert der Streuung der Normalseren (n=106). In erfindungsgemäßen Testsystem lag der Normwert nach dieser Berechnung bei einer aus der Standardkurve errechneten Konzentration von 223 Einheiten.

Abb. 1 zeigt die schematische Darstellung der Peptide und ihre Reaktivität bei den 40 SLE-Seren sowie bei den 50 gesunden Probanden.

Die Grenze zum Normalbereich wurde aus der mittleren Extinktion der 106 Blutspender plus dem zweifachen Wert der Streuung ermittelt. Unter Verwendung der relativ kurzen Peptide 83-99 und 95-119 werden im ELISA positive Reaktionen nur in jeweils 4% bzw. 25% der untersuchten Seren gemessen. Wird dagegen das Peptid mit der AS 83-119 als Festphasenantigen verwendet, so kommt es zum deutlichen Anstieg der positiven Reaktionen auf ca 70% aller untersuchten SLE-Seren (Abb. 1).

Überraschend zeigte sich, daß die Verwendung längerer Peptide als Festphasenantigen im ELISA eine deutliche qualitative Verbesserung gegenüber bisher beschriebenen Assays erbringt.

### Beispiel 2

### Untersuchungen über Spezifität und Sensivität des ELISAs mit dem Peptid 83-119

Es wurden insgesamt 176 SLE-Seren, 89 Seren von HIV-infizierten Patienten, 25 MCTD-Seren, 28 Seren von Patienten mit einer rheumatischen Arthritis, 15 Seren mit einem Sjögren-Syndrom, 20 Seren mit einer progressiven Sklerodermie (PSS), 30 Seren von Patienten mit einer Lyme-disease, 20 Hepatitis B-positive Patienten, 25 Seren mit Colitis ulcerosa und 105 Blutspender getestet.

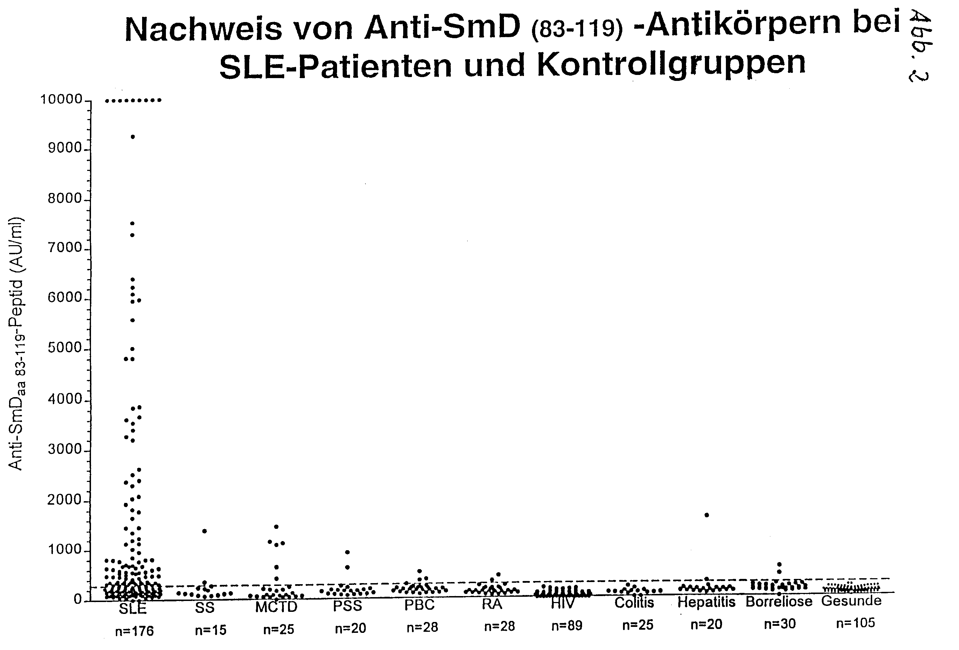

Antikörper gegen das Peptid 83-119 ließen sich in 67,3% aller untersuchten SLE-Seren nachweisen. Damit ist der ELISA hochspezifisch für den SLE. Vereinzelt treten positive Reaktionen auch bei der MCTD oder der RA auf, hohe Konzentrationen werden jedoch nahezu nur beim SLE erreicht (Abb. 2).

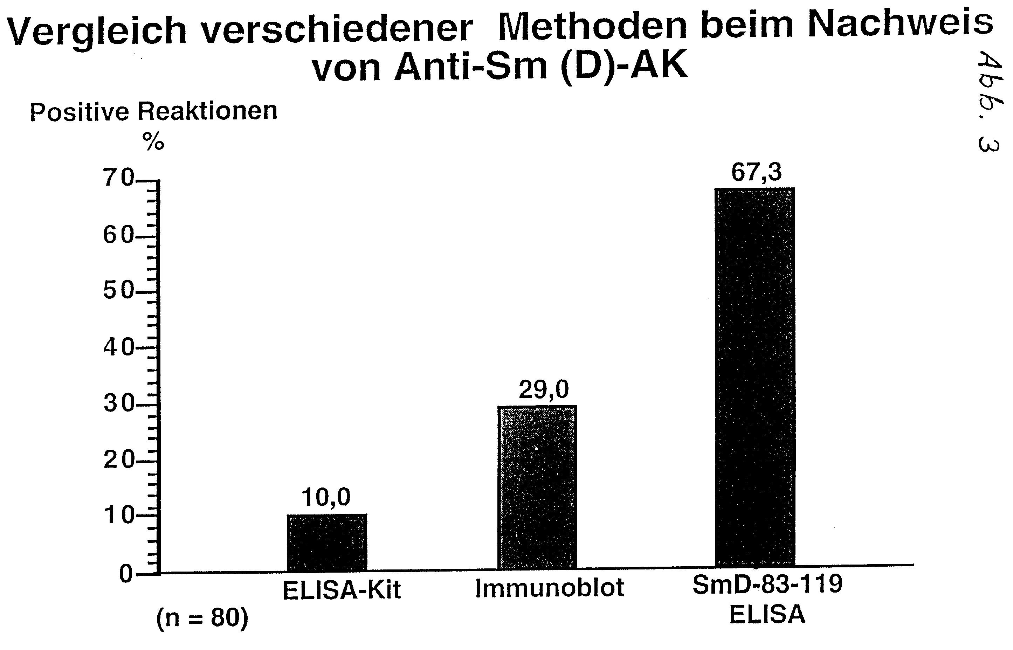

Bei Verwendung eines kommerziellen Anti-Sm-ELISA (Sm-Elias) ließen sich Anti-Sm-Ak nur in 10% der Seren nachweisen (Abb. 3).

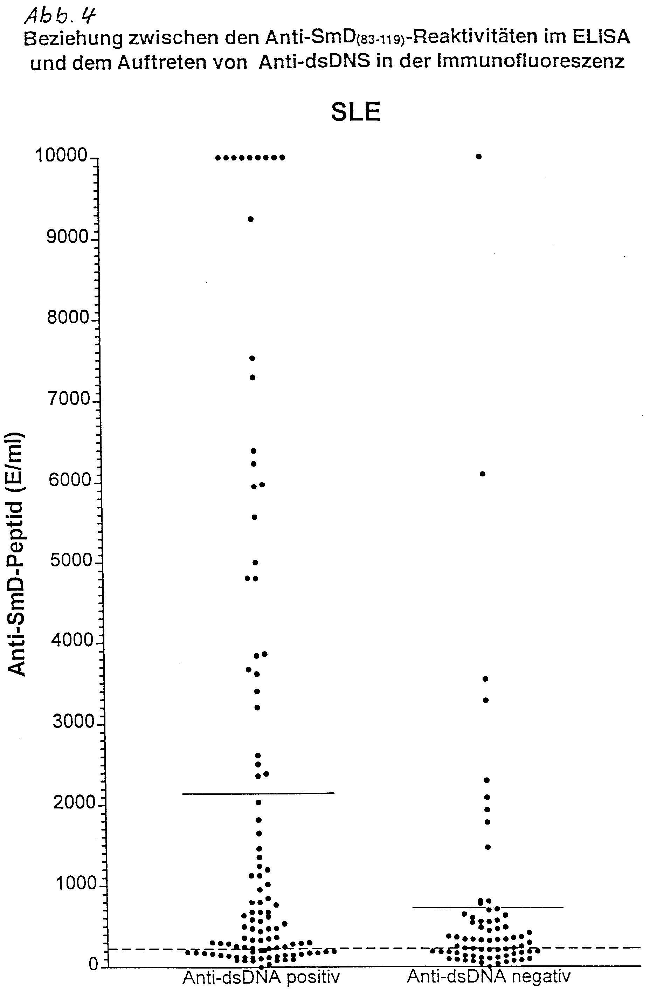

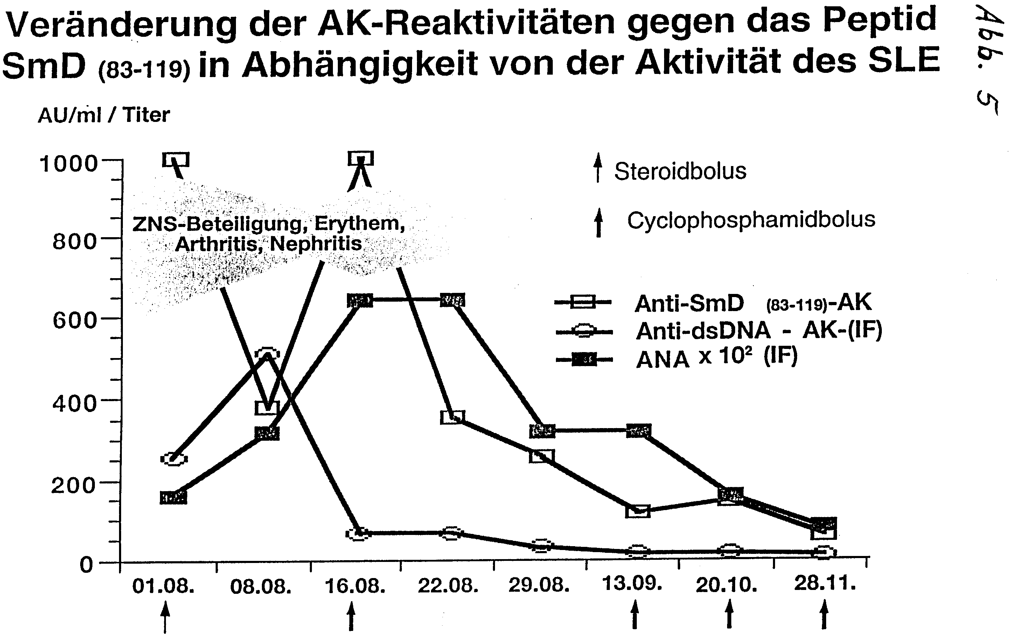

Vergleicht man SLE-Seren mit negativen Anti-dsDNA-Ak mit solchen mit Anti-dsDNA-Ak, so lassen sich die Antikörper gegen das SmD-Peptid in signifikant höheren Titern bei Anti-dsDNA-Ak-positiven Patienten nachweisen (Abb. 4). Die Reaktivitäten im ELISA scheinen weiterhin abhängig zu sein von der Krankheitsaktivität. Um diese Beobachtung zu überprüfen, wurden 6 Patienten im Verlauf untersucht. Die Abb. 5 zeigt den Verlauf einer typischen Patientin und den Einfluß der Therapie auf den Autoantikörper-Titer. Bei einer Patientin waren die Anti-Peptid-Antikörper nachweisbar, obwohl die Anti-dsDNA-Ak negativ waren.

Mit der Entwicklung dieses neuen Testes mit dem Peptid 83-119 an der festen Phase lassen sich Verlaufsbeurteilungen relativ einfach und schnell durchführen. Damit stellen sie eine gute Alternative zu den bisherigen aufwendigen Test dar.

### Beispiel 3

### Nachweis von Autoantikörpern gegen andere Autoantigene

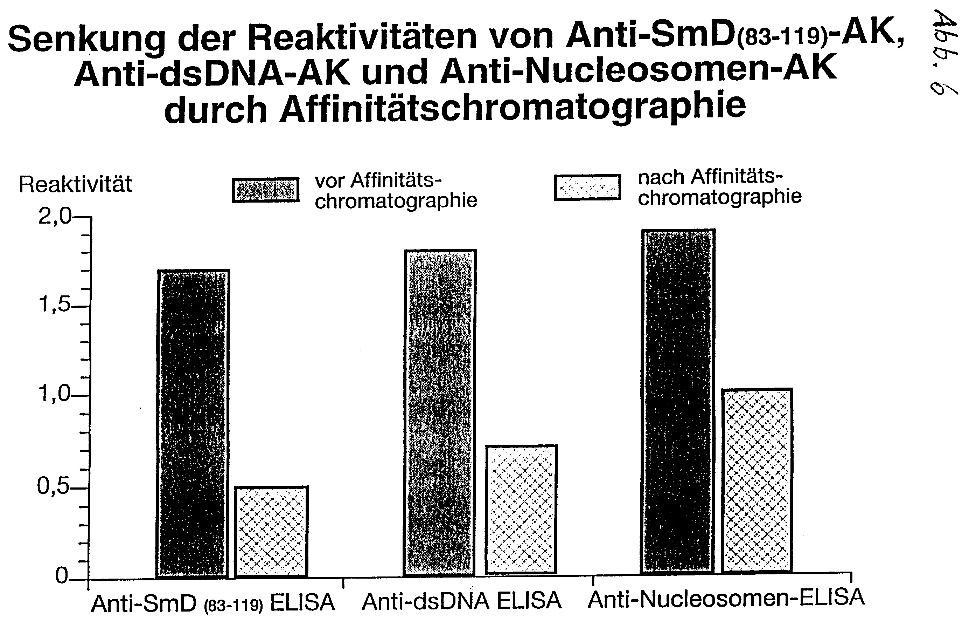

Die Häufigkeit der Anti-Peptid-Ak entspricht damit nahezu der von Anti-dsDNA-Ak. Kürzlich konnten Reichlin und Mitarbeiter eine Kreuzreaktivität der Anti-dsDNA-Ak mit dem Ak gegen das Sm-D und A-Protein des snRNP nachweisen (Reichlin, M.A. et al, J. Clin. Invest. 1994; 93: 443-449). Da nur eine geringe Homologie zwischen den Aminosäuren besteht, wurde die Ursache der Kreuzreaktionen auf mögliche Konformationsepitope zurückgeführt. Um eine mögliche Kreuzreaktivität der Anti-SmD₍₈₃₋₁₁₉₎-Ak mit anderen Autoantikörpern nachzuweisen, wurde eine Affinitätschromatographie mit dem an Sepharose gebundenen SmD₍₈₃₋₁₁₉₎-Peptid durchgeführt und die Extinktionen in unterschiedlichen ELISAs vor und nach Eluation der Anti-Peptid-Ak gemessen (Abb. 6). Es kam zum signifikanten Abfall sowohl im Anti-SmD-Peptid-ELISA als auch im ELISA zum Nachweis von Anti-dsDNA-Ak und Antikörpern gegen Nukleosomen. Zwischen diesen Autoantigenen scheinen demzufolge Kreuzreaktionen zu bestehen.

## Patentansprüche

1. Peptide des Antigens Sm-D, bestehend aus 35 bis 45 Aminosäuren, die ein Konformationsepitop bilden und Autoantikörper binden können, wie sie beim systemischen Lupus erythematodes (SLE) vorkommen.

2. Peptide gemäß Anspruch 1, dadurch gekennzeichnet, daß sie entweder ganz oder teilweise oder als Variante einer der folgenden Peptide aufgebaut sind:
a) PKVKSKKREAVAGRGRGRGRGRGRGRGRGRGGPRR
b) EPKVKSKKREAVAGRGRGRGRGRGRGRGRGRGGPRR
c) VEPKVKSKKREAVAGRGRGRGRGRGRGRGRGRGGPRR
d) DVEPKVKSKKREAVAGRGRGRGRGRGRGRGRGRGGPRR
e) VDVEPKVKSKKREAVAGRGRGRGRGRGRGRGRGRGGPRR
f) RVDVEPKVKSKKREAVAGRGRGRGRGRGRGRGRGRGGPRR
g) IRVDVEPKVKSKKREAVAGRGRGRGRGRGRGRGRGRGGPRR
h) TIRVDVEPKVKSKKREAVAGRGRGRGRGRGRGRGRGRGGPRR
i) DTIRVDVEPKVKSKKREAVAGRGRGRGRGRGRGRGRGRGGPRR
j) LDTIRVDVEPKVKSKKREAVAGRGRGRGRGRGRGRGRGRGGPRR
k) PLDTIRVDVEPKVKSKKREAVAGRGRGRGRGRGRGRGRGRGGPRR

3. Peptide gemäß Anspruch 1 und 2, gekennzeichnet durch die Struktur
VEPKVKSKKREAVAGRGRGRGRGRGRGRGRGRGGPRR
bzw. durch diese an einer oder mehreren beliebigen Stellen durch Austausch von Aminosäuren geänderte Struktur bzw. durch diese an einer oder mehreren beliebigen Stellen durch Entfernen von Aminosäuren geänderte Struktur.

4. Antikörper, die gegen Konformationsepitope des Sm-D-Antigens gerichtet und dadurch gekennzeichnet sind, daß sie ein oder mehrere Peptide der Ansprüche 1-3 erkennen.

5. Antikörper, die gegen Konformationsepitope des Sm-D-Antigens gerichtet und dadurch gekennzeichnet sind, daß sie das Peptid
VEPKVKSKKREAVAGRGRGRGRGRGRGRGRGRGGPRR
bzw. seine Mutanten und Varianten erkennen.

6. Anti-Idiotyp-Antikörper, welche gegen einen Antikörper gemäß Anspruch 4 gebildet werden und dadurch charakterisiert sind, daß sie auf spezifische Art die Autoantikörper des SLE in biologischen Flüssigkeiten erkennen.

7. Antigene Kompositionen, welche ein oder mehrere Peptide gemäß der Ansprüche 1-3 enthalten und dadurch charakterisiert sind, daß sie mit den Autoantikörpern, wie sie in biologischen Flüssigkeiten beim SLE vorkommen, spezifisch reagieren.

8. Antigene Kompositionen, welche das Peptid
VEPKVKSKKREAVAGRGRGRGRGRGRGRGRGRGGPRR
bzw. seine Mutanten und Varianten enthalten und dadurch charakterisiert sind, daS sie mit den Autoantikörpern, wie sie in biologischen Flüssigkeiten beim SLE vorkommen, spezifisch reagieren.

9. Immunogene Kompositionen, dadurch gekennzeichnet, daß sie ein Peptid gemäß den Ansprüchen 1-3 oder eine Verbindung dieses Peptids mit einem Trägermolekül enthalten und die Produktion der Antikörper, die fähig sind, Autoantigene beim SLE zu erkennen, induzieren.

10. Immunogene Kompositionen, dadurch gekennzeichnet, daß sie das Peptid VEPKVKSKKREAVAGRGRGRGRGRGRGRGRGRGGPRR bzw. seine Mutanten oder Varianten enthalten und die Produktion der Antikörper, die fähig sind, Autoantigene beim SLE zu erkennen, induzieren.

11. Verfahren zur Bestimmung von Anti-Sm-D-Antikörpern in biologischen Flüssigkeiten, dadurch gekennzeichnet, daß es mindestens die folgenden Schritte
- das in Kontaktbringen von biologischen Flüssigkeiten mit zumindest einem Peptid gemäß den Ansprüchen 1-3, oder einer Verbindung dieser Peptide mit einem Trägermolekül oder auch einem antiidiotypischen Antikörper gemäß Anspruch 6, unter solchen Bedingungen, die eine Antigen-Antikörper-Reaktion zulassen und
- den Nachweis dieser Antigen-Antikörper-Reaktion oder einer Antikörper-Anti-Idiotyp-Antikörper-Reaktion durch physikalische oder chemische Methoden
enthält.

12. Testkit für die Bestimmung von Anti-Sm-D-Antikörpern, gekennzeichnet durch
- mindestens 1 Peptid der Ansprüche 1-3 oder einer Verbindung dieses Peptids mit einem Trägermolekül oder auch ein Anti-Idiotyp-AK gemäß Anspruch 6, das adsorptiv oder kovalent an eine feste Phase gebunden ist,
- einen Puffer zur Verdünnung der zu untersuchenden biologischen Flüssigkeit, der nicht-ionische oder ionische Detergentien oder Proteine enthält,
- ein spezifisches Konjugat, bestehend aus einem Antikörper, der gegen humanes Immunglobulin IgG, IgM, IgA oder gegen mehrere dieser Immunglobuline gerichtet ist, und einem Enzym,
- einen Puffer zum Verdünnen des Konjugats, der nichtionische oder ionische Detergentien oder Proteine enthält,
- einen Puffer zum Waschen (Entfernen) der nichtgebundenen Immunreaktanten, der nicht-ionische oder ionische Detergentien oder Proteine enthält,
- eine Substratlösung zum Nachweis der Enzymreaktion und
- eine Stopp-Lösung zum Unterbrechen der Enzymreaktion.

13. Testkit nach Anspruch 12, gekennzeichnet durch das Peptid VEPKVKSKKREAVAGRGRGRGRGRGRGRGRGRGGPRR bzw. seine Mutanten oder Varianten.

14. Verwendung der Peptide und ihrer Folgeprodukte gemäß einem der Ansprüche 1-3 zur Produktion von Medikamenten gegen den SLE.

15. Verwendung der Peptide und ihrer Folgeprodukte gemäß einem der Ansprüche 1-3 als Vakzine zur Erzeugung von Toleranzmechanismen.
